# FASCICULE DE BREVET EUROPEEN

(11) **EP 0 451 873 B1**
(45) Date de publication et mention de la délivrance du brevet: **08.03.1995**
(21) Numéro de dépôt: 91105958.2
(22) Date de dépôt: 19.11.1987
(51) Int. Cl.: C07C 69/757, C07C 255/39, C07D 213/647, C07C 62/02

(54) **Nouveaux dérivés de l'acide 2,2-diméthyl cyclopropanecarboxylique portant en 3 une chaîne halogénée saturée, leur procédé de préparation et leur application à la synthèse de produits pesticides**
In stellung 3 durch eine halogenierte gesättigte Kette substituierte 2,2-dimethylcyclopropancarbonsäurederivate, Verfahren zu deren Herstellung und deren Verwendung zur Herstellung von pestiziden Produkten
Derivatives of 2,2-dimethyl cyclopropanecarboxylic acid substituted at c-3 by a saturated halogenated chain, process for their preparation and their use in the synthesis of pesticidal products

(30) Priorité: 20.11.1986 FR 8616155
(43) Date de publication de la demande: 16.10.1991
(62) Demande divisionnaire de: 87402605.7
(73) Titulaire: ROUSSEL-UCLAF, 75007 Paris (FR)
(72) Inventeur: Cadiergue, Joseph, F-93600 Aulnay sous Bois (FR); Demoute, Jean-Pierre, F-93360 Neuilly Plaisance (FR); Tessier, Jean, F-94300 Vincennes (FR)
(74) Mandataire: Tonnellier, Marie-José

(56) Documents cités:
- EP-A- 0 187 674
- EP-A- 0 217 342
- GB-A- 2 099 810

## Description

La présente invention concerne l'application de dérivés de l'acide 2,2-diméthyl cyclopropane carboxylique portant en 3 une chaîne halogénée saturée.

On connaissait des procédés de préparation de 3-(2-chloro 3-trifluoro 1-propényl 2,2-diméthyl cyclopropane carboxylates, à partir des 3-formyl 2,2-diméthyl cyclopropane carboxylates correspondants par addition de dérivés halogénés, acétylation et réduction, cf Tetrahedron Letters 1986 27 p. 2139-2142, et Chemical Abstracts, 1977, 86 n ° 139480a et également EP-A-217 342, qui est un document de l'état de la technique au titre de l'Article 54-3 et 54-4 CBE non pertinent pour l'apréciation de l'activité inventive.

L'invention a pour objet l'application des composés de formule (1) : 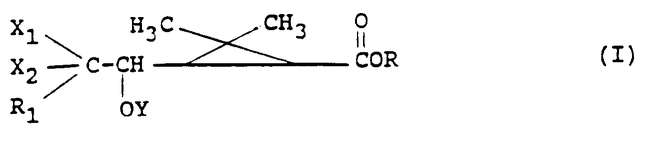 dans laquelle X₁ et X₂ identiques ou différents l'un de l'autre représentent un atome d'halogène, R₁ représente un atome d'halogène, un radical alkyle renfermant jusqu'à 8 atomes de carbone, un radical aryle renfermant jusqu'à 14 atomes de carbone éventuellement substitué, un radical perfluoroalkyle renfermant jusqu'à 8 atomes de carbone, un radical -C=N, un radical 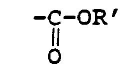 dans laquelle R' représente un radical alkyle renfermant jusqu'à 8 atomes de carbone, Y représente un radical choisi dans le groupe constitué par :
- les radicaux SO₂alc₁, alc₁ représentant un radical alcoyle linéaire ou ramifié, saturé ou insaturé éventuellement substitué par un ou plusieurs groupements fonctionnels ;
- les radicaux S0₂Aᵣ, Aᵣ représentant un radical aryle renfermant jusqu'à 14 atomes de carbone, éventuellement substitué par un ou plusieurs groupements fonctionnels ;
- les radicaux 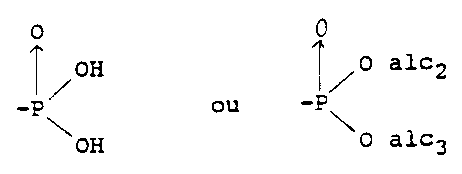 alc₂ et alc₃ identiques ou différents l'un de l'autre représentant un radical alcoyle linéaire ou ramifié saturé ou insaturé renfermant jusqu'à 8 atomes de carbone, éventuellement substitué par un ou plusieurs groupements fonctionnels ou alc₂ et alc₃
   pouvant former avec le radical 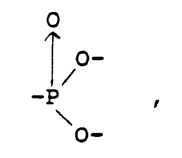 un cycle 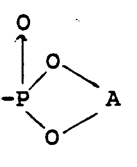 A représentant une chaîne carbonée linéaire ou ramifiée, saturée ou insaturée éventuellement substituée par un ou plusieurs groupements fonctionnels et renfermant jusqu'à 6 atomes de carbone ;
- les radicaux 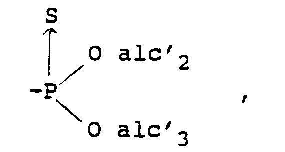 alc'₂ et alc'₃ représentant les mêmes valeurs que alc₂ et alc₃ ;
- le radical 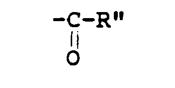 R" représentant un radical aryle et R représente
- soit un atome d'hydrogène,
- soit un radical alkyle, linéaire ou ramifié, éventuellement substitué renfermant de 1 à 8 atomes de carbone,
- soit un radical benzyle éventuellement substitué par un ou plusieurs radicaux choisis dans le groupe constitué par les radicaux alcoyles comportant de 1 à 4 atomes de carbone, les radicaux alcényles comportant de 2 à 6 atomes de carbone, les radicaux alcényloxy comportant de 2 à 6 atomes de carbone, les radicaux alcadiényles comportant de 4 à 8 atomes de carbone, le radical méthylènedioxy et les atomes d'halogène,
- soit un groupement : 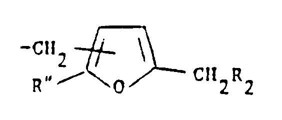 dans lequel le substituant R" représente un atome d'hydrogène ou un radical méthyle et le substituant R₂ un aryle monocyclique ou un groupement -C=CH et notamment un groupement 5-benzyl 3-furyl méthyle,
- soit un groupement : 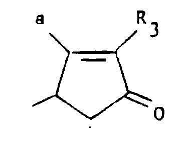 dans lequel a représente un atome d'hydrogène ou un radical méthyle et R₃ représente un radical organique aliphatique comportant de 2 à 6 atomes de carbone et une ou plusieurs insaturations carbone-carbone et notamment le radical -CH₂-CH=CH₂, -CH₂-CH=CH-CH₃, -CH₂-CH=CH-CH = CH₂, -CH₂-CH = CH-CH₂-CH₃, -CH2-C≡CH,
- soit un groupement : 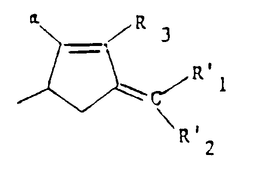 dans lequel a représente un atome d'hydrogène ou un radical méthyle, R₃ conserve la même signification que précédemment, R', et R'₂, identiques ou différents, représentent un atome d'hydrogène, un atome d'halogène, un radical alcoyle renfermant de 1 à 6 atomes de carbone, un radical aryle comportant de 6 à 10 atomes de carbone, un groupement alcoyloxycarbonyle comportant de 2 à 5 atomes de carbone, au un groupement cyano,
- soit un groupement : 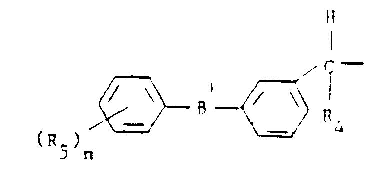 dans lequel B' représente un atome d'oxygène ou de soufre, un groupement 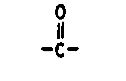 ou -CH₂ ou un groupement sulfoxyde ou un groupement sulfone et R₄ représente un atome d'hydrogène, un radical -C=N, un radical méthyle, un radical -CONH₂, un radical -CSNH₂ ou un radical -C=CH, R₅ représente un atome d'halogène ou un radical méthyle et n représente un nombre égal à 0,1 ou 2, et notamment le groupement 3-phénoxy benzyle, alpha-cyano 3-phénoxy benzyle, alpha-éthynyl 3-phénoxy benzyle, 3-benzoyl benzyle, 1-(3-phénoxy phényl) éthyl ou alpha-thioamido 3-phénoxy benzyle,
- soit un groupement : 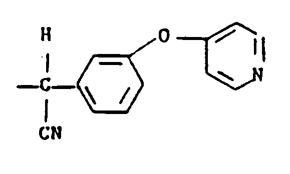
- soit un groupement : 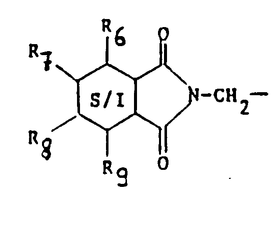 dans lequel les substituants R₆, R₇, R₈, R₉ représentent un atome d'hydrogène, un atome de chlore, ou un radical méthyle et dans lequel S/I symbolise un cycle aromatique ou un cycle analogue dihydro ou tétrahydro,
- soit un groupement : 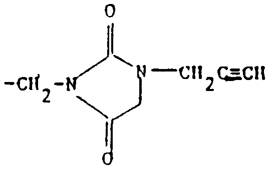
- soit un groupement : 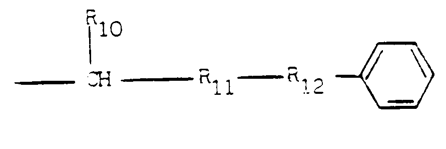 dans lequel R₁₀ représente un atome d'hydrogène ou un radical CN, R₁₂ représente un radical -CH₂ ou un atome d'oxygène, R₁₁ représente un radical thiazolyle ou thiadiazolyle dont la liaison avec 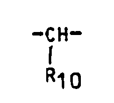 peut se trouver à l'une quelconque des positions disponibles, R₁₂ étant lié à R₁₁ par l'atome de carbone compris entre l'atome de soufre et un atome d'azote,
- soit un groupement : 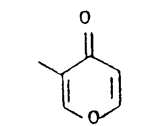
- soit un groupement : 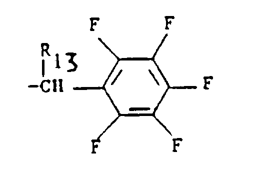 dans lequel R₁₃ représente un atome d'hydrogène ou un radical CN,
- soit un groupement : 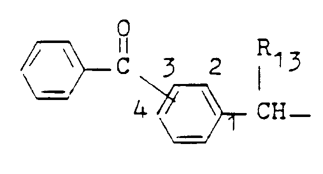 dans lequel R_{13 3} est défini comme ci-dessus, et le radical benzoyle est en position 3 ou 4,
- soit un groupement : 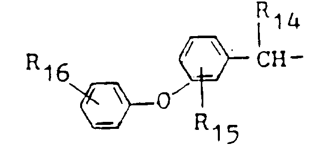 dans lequel R₁₄ représente un atome d'hydrogène, un radical méthyle, éthynyle ou cyano et R₁₅ et R₁₆,différents, représentent un atome d'hydrogène, de fluor ou de brome,
- soit un groupement : 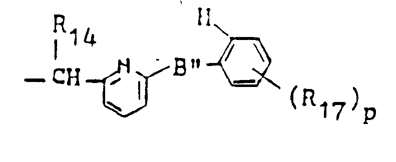 dans lequel R₁₄ est défini comme ci-dessus, chacun des R₁₇ représente indépendamment un groupement alcoyle renfermant de 1 à 4 atomes de carbone, alcoxy renfermant de 1 à 4 atomes de carbone, alcoylthio renfermant de 1 à 4 atomes de carbone, alcoylsulfonyl renfermant de 1 à 4 atomes de carbone, trifluorométhyl, 3,4-méthylène dioxy, chloro, fluoro ou brome, p représente un nombre égal à 0,1 ou 2 et B" représente un atome d'oxygène ou un atome de soufre,
- soit un groupement : 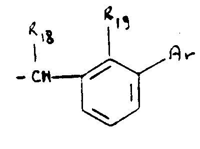 dans lequel R₁₈ représente un atome d'hydrogène, un radical méthyle, éthynyle ou cyano et R₁₉, différent, représente un atome d'hydrogène, de fluor ou de brome et Ar représente un radical aryle renfermant jusqu'à 14 atomes de carbone,
- soit un groupement :

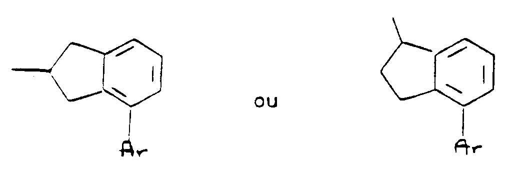

- soit un groupement :

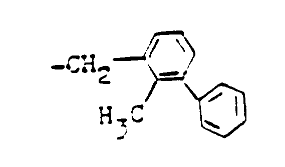

- soit un groupement : 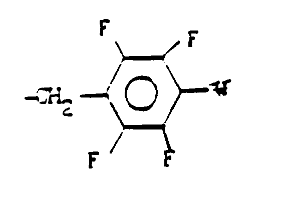 avec W = H, CH₃, CCH₃ à la condition que R₁ ne représente pas un radical CF₃ si R=H alkyle, à l'exception du 2,2-diméthyl-3-(1-méthane sulfonyloxy-2,2-dichloro-3,3,3-trifluoropropyl)-cyclopropane carboxylate de (2 méthyl-3-phényl phényl) méthyle, du 2,2-diméthyl-3-(1-tosyl oxy-2,2-dichloro-3,3,3-trifluoroptopyl cyclopropane carboxylate de (2-méthyl-3-phényl phényl) méthyle et du 2,2-diméthyl-3-(1-benzoyloxy-2,2-dichloro-3,3,3-trifluopropyl cyclopropane carboxylate de (2-méthyl-3-phényl phényl) méthyle, caractérisé en ce que l'on soumet un composé de formule (I) défini ci-dessus à l'action d'un agent de réduction pour obtenir le composé de formule (III) correspondant 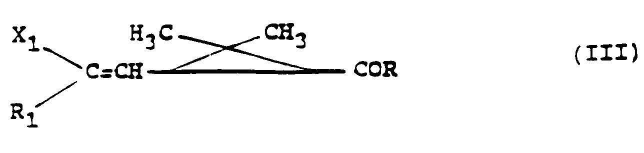 dans laquelle Xi, R₁ et R conservent leur signification précédente.

La préparation des composés de formule I est décrite et revendiquée dans le brevet européen 269514.

Les composés de formule 1 présentent plusieurs centres d'asymétrie, tes carbones en 1 et 3 du cyclopropane et les carbones en 1' et 2' de la chaîne latérale 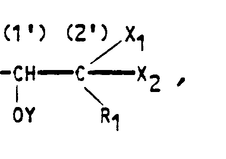 ils peuvent présenter également plusieurs centres d'asymétrie dans la partie R. L'invention a pour objet les différents stéréoisomères possibles ainsi que les mélanges de ces stéréoisomères.

X₁ et X₂ identiques ou différents représentent de préférence un atome de chlore, de brome ou d'iode.

Lorsque R₁ représente un atome d'halogène, il s'agit de préférence d'un atome de brome, de chlore ou de fluor.

Lorsque R₁ représente un radical alkyle, il s'agit de préférence du radical méthyle, éthyle, n-propyle, isopropyle, n-butyle, isobutyle ou terbutyle.

Lorsque R₁ représente un radical aryle, il s'agit de préférence du radical phényle.

Lorsque R₁ représente un radical aryle substitué, il s'agit de préférence du radical phényle substitué par un atome d'halogène, par exemple un atome de chlore.

Lorsque R₁ représente un radical perfluoroalkyle, il s'agit de préférence du radical CF₃.

Lorsque R₁ représente un radical 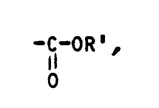 R' représente de préférence un radical méthyle, éthyle ou n-propyle, isopropyle, t-butyle ou hexafluoroiso- propyle.

Comme combinaisons préférées de X₁, X et R₁, on peut citer les combinaisons suivantes : 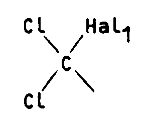
avec Hal₁ = Br, I 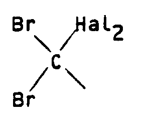
avec Hal₂ = Br, I 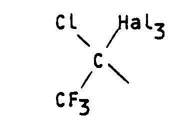
avec Hal₃ = Br, I 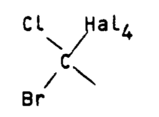
avec Hal₄ = Br, I 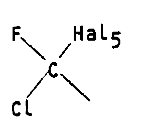
avec Halₛ = Br, I 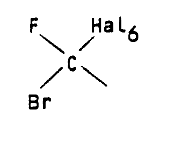
avec Hal₆ = Br,

Alc₁, alc₂, alc₃, alc'₂ et alc'₃ représentent de préférence un radical méthyle, éthyle, n-propyle, isopropyle, n-butyle, isobutyle, t-butyle.

Ar représente de préférence un radical phényle.

Lorsque alc₁, alc₂, alc₃, alc'₂, alc'₃ et Ar sont substitués, ils le sont de préférence par un ou plusieurs des substituants suivants : les atomes d'halogène, les radicaux CF₃, hydroxyle, carboxyle, amino, ammonium, les radicaux alkyle et alkoxy renfermant jusqu'à 4 atomes de carbone, par exemple les radicaux méthyle et méthoxy.

A représente de préférence une chaîne carbonée saturée renfermant de 1 à 4 atomes de carbone, éventuellement substituée par un ou plusieurs radicaux choisis dans le groupe suivant ; les radicaux alkyles renfermant de 1 à 5 atomes de carbone, les atomes d'halogéne, les radicaux CF₃ ou les radicaux hydroxyles.

Lorsque R représente un radical alkyle, il s'agit de préférence du radical méthyle, éthyle, n-propyle, isopropyle, butyle, isobutyle, terbutyle.

Les valeurs préférées des alcools utilisés dans la synthèse des esters pyréthrinoides seront indiquées ci-après.

L'invention a plus particulièrement pour objet l'application des composés dans lesquels Y représente un radical SO₂alc₁ , alc₁ représentant un radical alkyle ou alkényle renfermant jusqu'à 8 atomes de carbone par exemple un radical S0₂CH₃.

Dans un mode de réalisation préférée, l'agent de réduction utilisé est l'hydrogène en présence d'un catalyseur d'hydrogénation comme le palladium. On peut également effectuer cette réduction avec du zinc en présence d'un acide. On peut aussi utiliser le couple zinc-cuivre au sein d'un alcool ou toute autre méthode permettant d'éliminer simultanément un atome d'halogène et le groupe OY.

Comme application, particulièrement intéressante, on peut citer l'application caractérisée en ce que le composé de formule 1 répond à la formule l' : 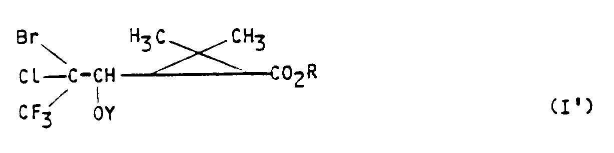 dans laquelle Y et R conservent la même signification que précédemment et le composé de formule III à la formule III' : 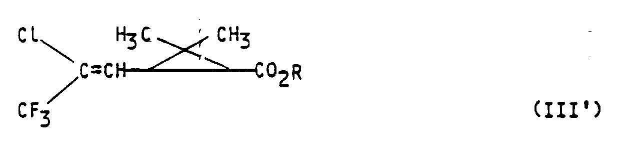 ou encore l'application caractérisée en ce que le composé de formule 1 répond à la formule 1" : 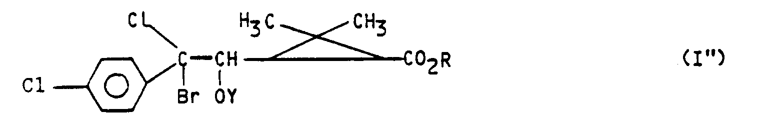 dans laquelle Y et R conservent la même signification que précédemment et le composé III obtenu à la formule III" :

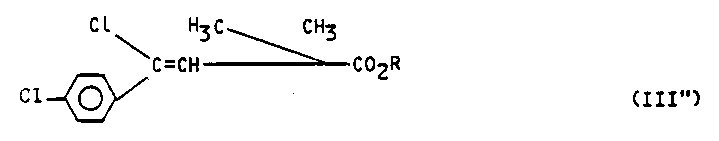

L'exemple suivant illustre l'invention sans toutefois la limiter.

### Acide [1R-[1-alpha,3-alpha]] 2,2-diméthyl 3-[2-trifluoromethyl 2-chloro éthényl] cyclopropane carboxylique.

On hydrogène 2g de [1R-[1-alpha, 3-alpha-(RS*,RS*)]] 2,2-diméthyl 3-[2-trifluorométhyl 2-bromo 2-chloro 1-méthyl sulfonyloxyéthyl) cyclopropane carboxylate de (3-phénoxyphényl) méthyle dans 30 cm3 d'éthanol en présence de catalyseur, 200 mg de palladium à 10%. On filtre, amène le filtrat à sec sous pression réduite. On obtient ainsi le produit que l'on dissout dans 20 cm3 d'une solution de soude normale. On lave la phase aqueuse au chlorure de méthylène, acidifie à l'acide chlorhydrique concentré et extrait au chlorure de méthylène. On sèche et amène sec. On obtient ainsi 400 mg de produit recherché fondant à 103◊C.

### Spectre RMN CDCl₃

### Isomère Z Ha = 6,83 6,98 ppm

### Isomère DE = 6.55 6.7 ppm

La préparation du produit de départ de cet exemple est décrite dans le brevet 269514.

## Revendications

1. Application des composés de formule (1) : 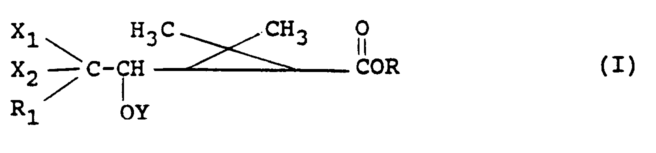 dans laquelle X₁ et X₂ identiques ou différents l'un de l'autre représentent un atome d'halogène, R₁ représente un atome d'halogène, un radical alkyle renfermant jusqu'à 8 atomes de carbone, un radical aryle renfermant jusqu'à 14 atomes de carbone éventuellement substitué, un radical perfluoroalkyle renfermant jusqu'à 8 atomes de carbone, un radical -C=N, un radical 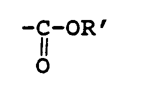 dans laquelle R' représente un radical alkyle renfermant jusqu'à 8 atomes de carbone, Y représente un radical choisi dans le groupe constitué par :
- les radicaux SO₂alc₁, alc₁ représentant un radical alcoyle linéaire ou ramifié, saturé ou insaturé éventuellement substitué par un ou plusieurs groupements fonctionnels ;
- les radicaux S0₂Aᵣ, Aᵣ représentant un radical aryle renfermant jusqu'à 14 atomes de carbone, éventuellement substitué par un ou plusieurs groupements fonctionnels ;
- les radicaux 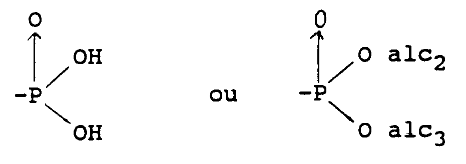 alc₂ et alc₃ identiques ou différents l'un de l'autre représentant un radical alcoyle linéaire ou ramifié saturé ou insaturé renfermant jusqu'à 8 atomes de carbone, éventuellement substitué par un ou plusieurs groupements fonctionnels ou alc₂ et alc₃ pouvant former avec le radical 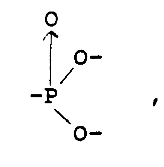 un cycle 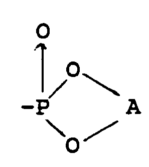 A représentant une chaîne carbonée linéaire ou ramifiée, saturée ou insaturée éventuellement substituée par un ou plusieurs groupements fonctionnels et renfermant jusqu'à 6 atomes de carbone ;
- les radicaux 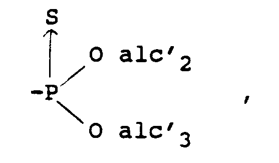 alc'₂ et alc'₃ représentant les mêmes valeurs que alc₂ et alc₃ ;
- le radical 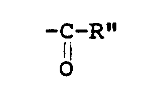 R" représentant un radical aryle et R représente
- soit un atome d'hydrogène,
- soit un radical alkyle, linéaire ou ramifié, éventuellement substitué renfermant de 1 à 8 atomes de carbone,
- soit un radical benzyle éventuellement substitué par un ou plusieurs radicaux choisis dans le groupe constitué par les radicaux alcoyles comportant de 1 à 4 atomes de carbone, les radicaux alcényles comportant de 2 à 6 atomes de carbone, les radicaux alcényloxy comportant de 2 à 6 atomes de carbone, les radicaux alcadiényles comportant de 4 à 8 atomes de carbone, le radical méthylènedioxy et les atomes d'halogène,
- soit un groupement : 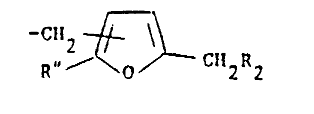 dans lequel le substituant R" représente un atome d'hydrogène ou un radical méthyle et le substituant R₂ un aryle monocyclique ou un groupement -C=CH et notamment un groupement 5-benzyl 3-furyl méthyle,
- soit un groupement : 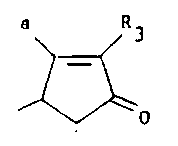 dans lequel a représente un atome d'hydrogène ou un radical méthyle et R₃ représente un radical organique aliphatique comportant de 2 à 6 atomes de carbone et une ou plusieurs insaturations carbone-carbone et notamment le radical -CH₂-CH = CH₂, -CH₂-CH = CH-CH₃, -CH₂-CH = CH-CH = CH₂, -CH₂-CH = CH-CH₂-CH₃, -CH2-C≡CH,
- soit un groupement : 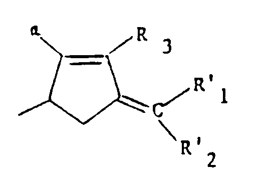 dans lequel a représente un atome d'hydrogène ou un radical méthyle, R₃ conserve la même signification que précédemment, R'₁ et R'₂, identiques ou différents, représentent un atome d'hydrogène, un atome d'halogène, un radical alcoyle renfermant de 1 à 6 atomes de carbone, un radical aryle comportant de 6 à 10 atomes de carbone, un groupement alcoyloxycarbonyle comportant de 2 à 5 atomes de carbone, ou un groupement cyano,
- soit un groupement : 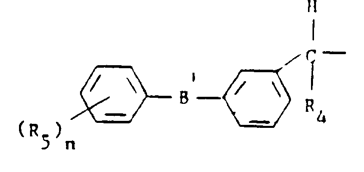 dans lequel B' représente un atome d'oxygène ou de soufre, un groupement 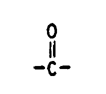 ou -CH₂ ou un groupement sulfoxyde ou un groupement sulfone et R₄ représente un atome d'hydrogène, un radical -C=N, un radical méthyle, un radical -CONH₂, un radical -CSNH₂ ou un radical -C=CH, R₅ représente un atome d'halogène ou un radical méthyle et n représente un nombre égal à 0,1 ou 2, et notamment le groupement 3-phénoxy benzyle, alpha-cyano 3-phénoxy benzyle, alpha-éthynyl 3-phénoxy benzyle, 3-benzoyl benzyle, 1-(3-phénoxy phényl) éthyl ou alpha-thioamido 3-phénoxy benzyle,
- soit un groupement : 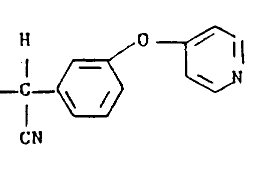
- soit un groupement : 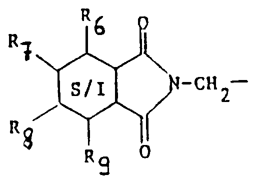 dans lequel les substituants R₆, R₇, R₈, R₉ représentent un atome d'hydrogène, un atome de chlore, ou un radical méthyle et dans lequel S/I symbolise un cycle aromatique ou un cycle analogue dihydro ou tétrahydro,
- soit un groupement : 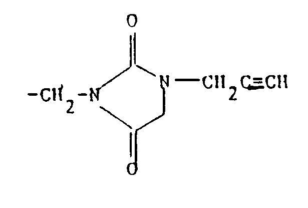
- soit un groupement : 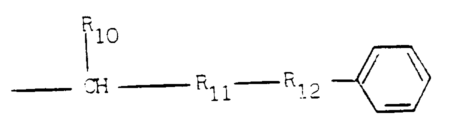 dans lequel R₁₀ représente un atome d'hydrogène ou un radical CN, R₁₂ représente un radical
-CH₂ où un atome d'oxygène, R₁₁ représente un radical thiazolyle ou thiadiazolyle dont la liaison avec 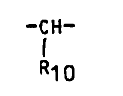 peut se trouver à l'une quelconque des positions disponibles, R₁₂ étant lié à R₁₁ par l'atome de carbone compris entre l'atome de soufre et un atome d'azote,
- soit un groupement : 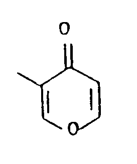
- soit un groupement : 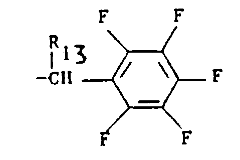 dans lequel R₁₃ représente un atome d'hydrogène ou un radical CN,
- soit un groupement : 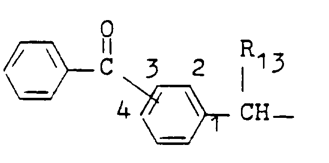 dans lequel R_{13 3} est défini comme ci-dessus, et le radical benzoyle est en position 3 ou 4,
- soit un groupement : 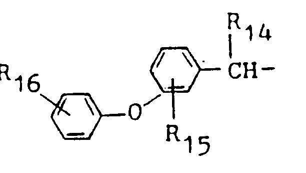 dans lequel R₁₄ représente un atome d'hydrogène, un radical méthyle, éthynyle ou cyano et R₁₅ et R₁₆, différents, représentent un atome d'hydrogène, de fluor ou de brome,
- soit un groupement : 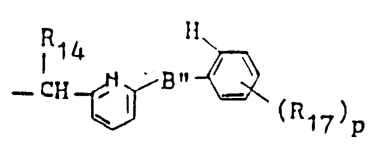 dans lequel R₁₄ est défini comme ci-dessus, chacun des R₁₇ représente indépendamment un groupement alcoyle renfermant de 1 à 4 atomes de carbone, alcoxy renfermant de 1 à 4 atomes de carbone, alcoylthio renfermant de 1 à 4 atomes de carbone, alcoylsulfonyl renfermant de 1 à 4 atomes de carbone, trifluorométhyl, 3,4-méthylène dioxy, chloro, fluoro ou brome, p représente un nombre égal à 0,1 ou 2 et B" représente un atome d'oxygène ou un atome de soufre,
- soit un groupement : 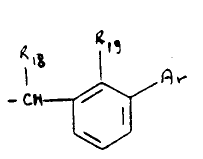 dans lequel R₁₈ représente un atome d'hydrogène, un radical méthyle, éthynyle ou cyano et Ri 9, différent, représente un atome d'hydrogène, de fluor ou de brome et Ar représente un radical aryle renfermant jusqu'à 14 atomes de carbone,
- soit un groupement : 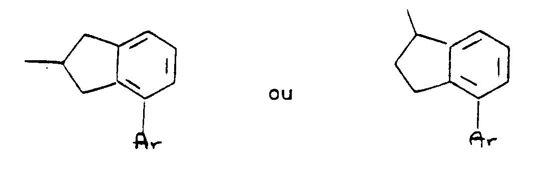
- soit un groupement : 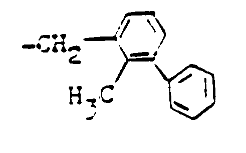
- soit un groupement : 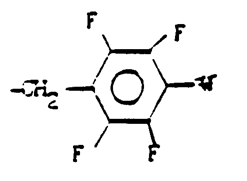 avec W = H, CH₃, CCH₃ à la condition que R₁ ne représente pas un radical CF₃ si R=H alkyle, à l'exception du 2,2-diméthyl-3-(1-méthane sulfonyloxy-2,2-dichloro-3,3,3-trifluoropropyl)-cyclopropane carborylate de (2 méthyl-3-phényl phényl) méthyle, du 2,2-diméthyl-3-(1-tosyl oxy-2,2-dichloro-3,3,3-trifluoropropyl cyclopropane carboxylate de (2-méthyl-3-phényl phényl) méthyle et du 2,2-diméthyl-3-(1-benzoyloxy-2,2-dichloro-3,3,3-trifluopropyl cyclopropane carboxylate de (2-méthyl-3-phényl phényl) méthyle, caractérisé en ce que l'on soumet un composé de formule (I) défini ci-dessus à l'action d'un agent de réduction pour obtenir le composé de formule (III) correspondant 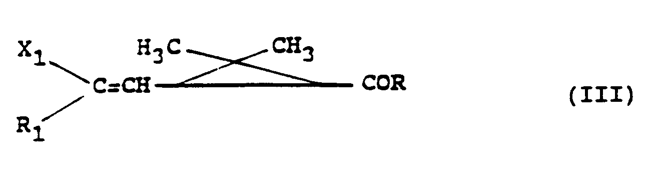 dans laquelle Xi, R₁ et R conservent leur signification précédente.

2. Application selon la revendication 1, caractérisée en ce que Y represente un radical SO₂alc₁, alc₁ représentant un radical alkyle ou alkényle renfermant jusqu'à 8 atomes de carbone.

3. Application selon la revendication 2, caractérisée en ce que alc₁ représente un radical méthyle.

4. Application selon l'une quelconque des revendications 1 à 3, caractérisée en ce que l'agent de réduction est l'hydrogène en présence d'un catalyseur d'hydrogénation comme le palladium.

5. Application selon l'une quelconque des revendications 1 à 4, caractérisée en ce que le composé de formule (I) répond à la formule (l') : 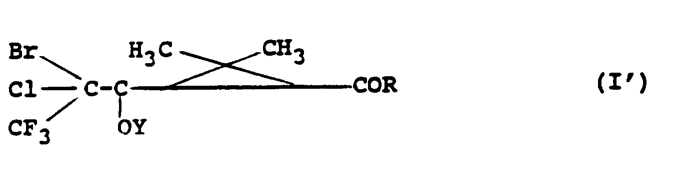 dans laquelle Y et R conservent leur signification précédente et le composé de formule (III) à la formule (III') :

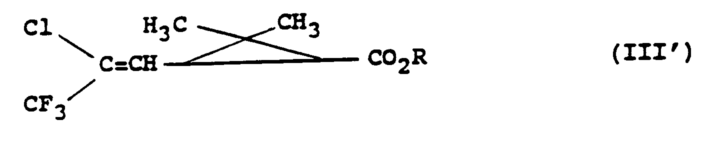

6. Application selon l'une quelconque des revendications 1 à 4, caractérisée en ce que le composé de formule (1) répond à la formule (I") : 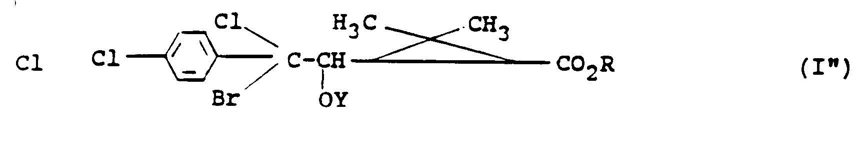 et le composé de formule (III) obtenu à la formule (III") :

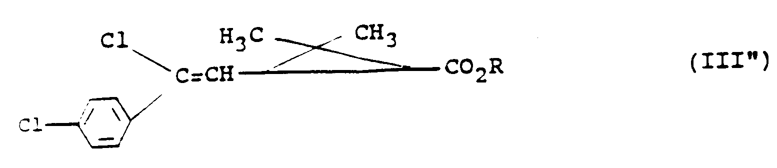

## Claims

1. Use of the compounds of formula (I): 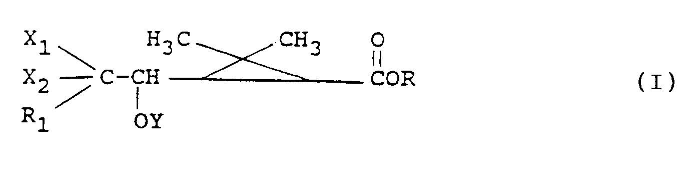 in which X₁ and X₂, identical to or different from one another, represent a halogen atom, R₁ represents a halogen atom, an alkyl radical containing up to 8 carbon atoms, an optionally substituted aryl radical containing up to 14 carbon atoms, a perfluoroalkyl radical containing up to 8 carbon atoms, a -C=N radical, a 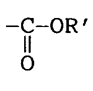 radical in which R' represents an alkyl radical containing up to 8 carbon atoms, Y represents a radical chosen from the group constituted by:
- the SO₂alk₁ radicals, alk₁ represents a saturated or unsaturated, linear or branched alkyl radical optionally substituted by one or more functional groups;
- the S0₂Aᵣ radicals, Aᵣ representing an aryl radical containing up to 14 carbon atoms, optionally substituted by one or more functional groups;
- the 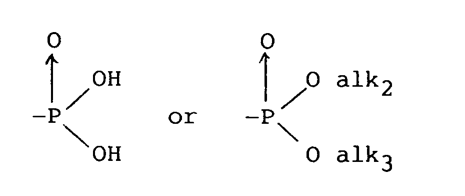 radicals alk₂ and alk₃, identical to or different from one another, representing a saturated or unsaturated, linear or branched alkyl radical containing up to 8 carbon atoms, optionally substituted by one or more functional groups or alk₂ and alk₃ which can form with the 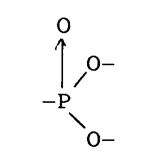 radical, a 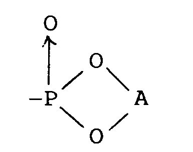 ring A representing a saturated or unsaturated, linear or branched carbonated chain optionally substituted by one or more functional groups and containing up to 6 carbon atoms;
- the 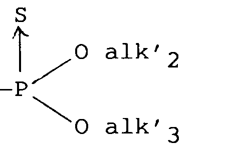 radicals, alk'₂ and alk'₃ representing the same values as alk₂ and alk₃:
- the 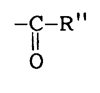 radical R" representing an aryl radical and R represents
- either a hydrogen atom,
- or an optionally substituted, linear or branched alkyl radical containing 1 to 8 carbon atoms,
- or a benzyl radical optionally substituted by one or more radicals chosen from the group constituted by alkyl radicals containing 1 to 4 carbon atoms, alkenyl radicals containing 2 to 6 carbon atoms, alkenyloxy radicals containing 2 to 6 carbon atoms, alkadienyl radicals containing 4 to 8 carbon atoms, the methylenedioxy radical and halogen atoms,
- or a group: 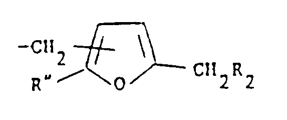 in which the substituent R" represents a hydrogen atom or a methyl radical and the substituent R₂ represents a monocyclic aryl or a -C=CH group, in particular a 5-benzyl 3-furyl methyl group,
- or a group: 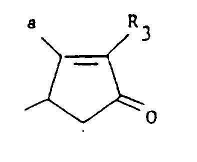 in which a represents a hydrogen atom or a methyl radical and R₃ represents an aliphatic organic radical containing 2 to 6 carbon atoms and one or more carbon-carbon unsaturations and in particular the -CH₂-CH = CH₂, -CH₂-CH = CH-CH₃, -CH₂-CH = CH-CH = CH₂, -CH₂-CH = CH-CH₂-CH₃, -CH₂-C≡CH radical,
- or a group: 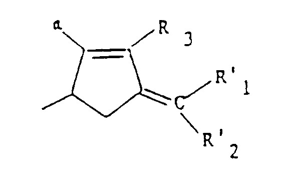 in which a represents a hydrogen atom or a methyl radical, R₃ retains the same meaning as previously, R'i and R'₂, identical or different, represent a hydrogen atom, a halogen atom, an alkyl radical containing 1 to 6 carbon atoms, an aryl radical containing 6 to 10 carbon atoms, an alkyloxycarbonyl group containing 2 to 5 carbon atoms, or a cyano group,
- or a group: 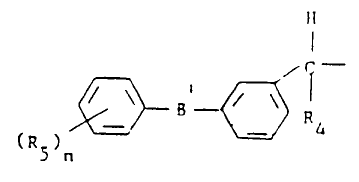 in which B' represents an oxygen or sulphur atom, a 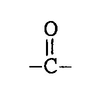 or -CH₂ group or a sulphoxide group or a sulphone group and R₄ represents a hydrogen atom, a -C=N radical, a methyl radical, a -CONH₂ radical, a -CSNH₂ radical or a -C=CH radical, R₅ represents a halogen atom or a methyl radical and n represents a number equal to 0, 1 or 2, and in particular the 3-phenoxy benzyl, alpha-cyano 3-phenoxy benzyl, alpha-ethynyl 3-phenoxy benzyl, 3-benzoyl benzyl, 1-(3-phenoxy phenyl) ethyl or alpha-thioamido 3-phenoxy benzyl group,
- or a group: 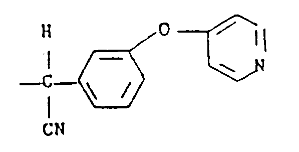
- or a group: 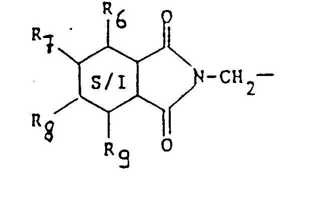 in which the substituents R₆, R₇, R₈, Rg represent a hydrogen atom, a chlorine atom or a methyl radical and in which S/I symbolises an aromatic ring or an analogous dihydro or tetrahydro ring,
- or a group: 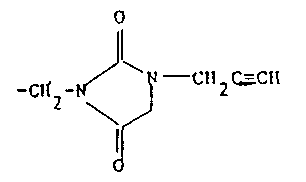
- or a group: 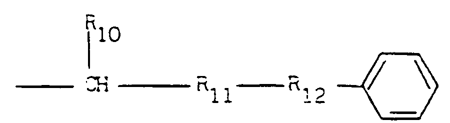 in which R₁₀ represents a hydrogen atom or a CN radical, R₁₂ represents a -CH₂ radical or an oxygen atom, R₁₁ represents a thiazolyl or thiadiazolyl radical of which the bond with 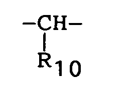 can be found in any one of the available positions, R₁₂ being linked to R₁₁ by the carbon atom contained between the sulphur atom and a nitrogen atom,
- or a group 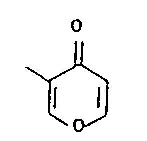
- or a group: 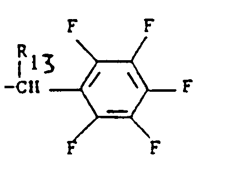 in which R₁₃ represents a hydrogen atom or a CN radical,
- or a group: 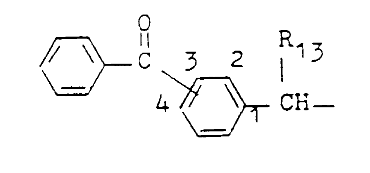 in which R_{13 3} is defined as above and the benzoyl radical is in position 3 or 4,
- or a group: 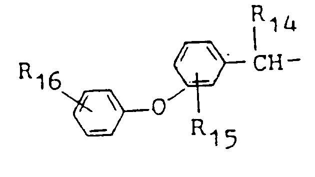 in which R₁₄ represents a hydrogen atom, a methyl, ethynyl or cyano radical, and R₁₅ and R₁₆, being different, represent a hydrogen, fluorine or bromine atom,
- or a group: 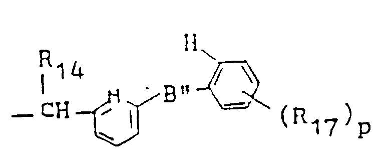 in which R₁₄ is defined as above, each of the R₁₇'s independently represent one of the following groups: alkyl containing 1 to 4 carbon atoms, alkoxy containing 1 to 4 carbon atoms, alkylthio containing 1 to 4 carbon atoms, alkylsulphonyl containing 1 to 4 carbon atoms, trifluoromethyl, 3,4-methylene dioxy, chloro, fluoro or bromine, p represents a number equal to 0, 1 or 2 and B" represents an oxygen atom or a sulphur atom,
- or a group 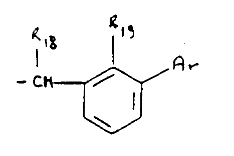 in which R₁₈ represents a hydrogen atom, a methyl, ethynyl or cyano radical and R₁₉, being different, represents a hydrogen, fluorine or bromine atom and Ar represents an aryl radical containing up to 14 carbon atoms
- or a group: 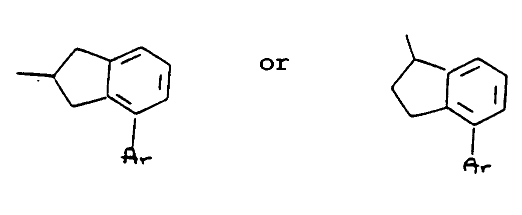
- or a group: 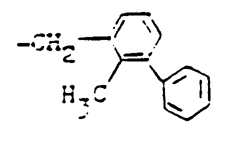
- or a group: 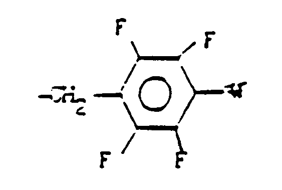 with W = H, CH₃, CCH₃ on the condition that R₁ does not represent a CF₃ radical if R=H alkyl, with the exception of (2 methyl-3-phenyl phenyl) methyl 2,2-dimethyl-3-(1-methane sulphonyloxy-2,2-dichloro-3,3,3-trifluoropropyl)-cyclopropane carboxylate, (2 methyl-3-phenyl phenyl) methyl 2,2-dimethyl-3-(1-tosyl oxy-2,2-dichloro-3,3,3-trifluoropropyl) cyclopropane carboxylate and (2 methyl-3-phenyl phenyl) methyl 2,2-dimethyl-3-(1-benzoyloxy-2,2-dichloro-3,3,3-trifluoropropyl) cyclopropane carboxylate, characterized in that a compound of formula (I) defined above is subjected to the action of a reducing agent in order to obtain the corresponding compound of formula (III): 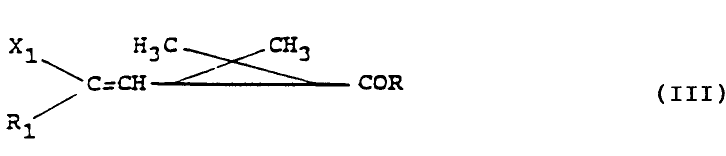 in which Xi, R₁ and R retain their previous meaning.

2. Use according to claim 1, characterized in that Y represents an SO₂alk₁ radical, alk₁ representing an alkyl or alkenyl radical containing up to 8 carbon atoms.

3. Use according to claim 2, characterized in that alk₁ represents a methyl radical.

4. Use according to any one of claims 1 to 3, characterized in that the reducing agent is hydrogen in the presence of a hydrogenation catalyst such as palladium.

5. Use according to any one of claims 1 to 4, characterized in that the compound of formula (I) corresponds to formula (I'): 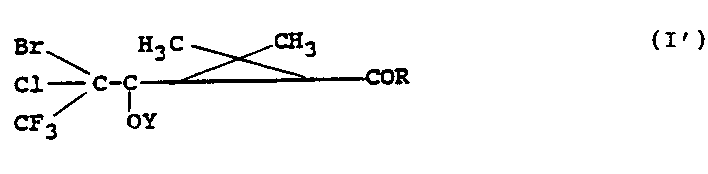 in which Y and R retain their previous meaning and the compound of formula (III) corresponds to formula (III'):

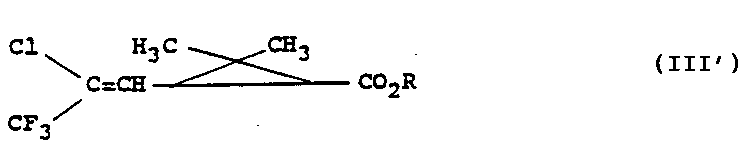

6. Use according to any one of claims 1 to 4, characterized in that the compound of formula (I) corresponds to formula (I"): 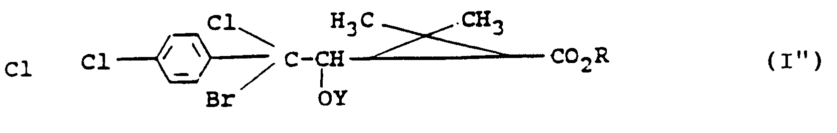 and the compound of formula (III) obtained corresponds to formula (III"):

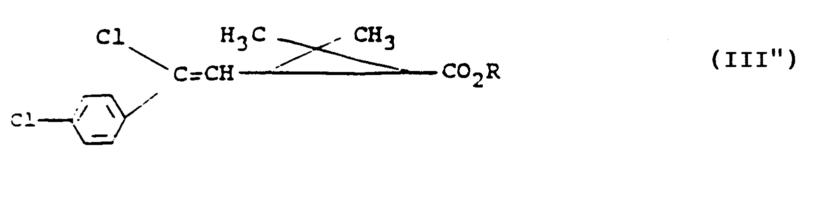

## Patentansprüche

1. Verwendung der Verbindungen der Formel (I) 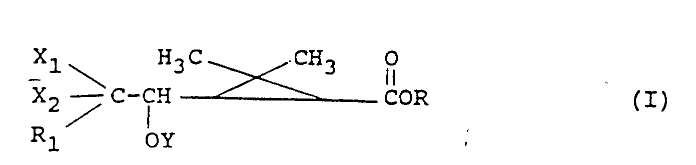 worin X₁ und X₂, die gleich oder verschieden voneinander sind, für ein Halogenatom stehen, R₁ für ein Halogenatom, einen Alkylrest mit bis zu 8 Kohlenstoffatomen, einen Arylrest mit bis zu 14 Kohlenstoffatomen, der gegebenenfalls substituiert ist, einen Perfluoralkylrest mit bis zu 8 Kohlenstoffatomen, einen -C=N-Rest, einen Rest 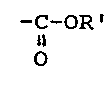 worin R' für einen Alkylrest mit bis zu 8 Kohlenstoffatomen steht, steht, Y für einen Rest, ausgewählt aus der Gruppe bestehend aus:
- SO₂alc₁-Resten, wobei alc₁ für einen linearen oder verzweigten, gesättigten oder ungesättigten, gegebenenfalls durch eine oder mehrere funktionelle Gruppen substituierten Alkylrest steht;
- S0₂Aᵣ-Resten, worin Aᵣ für einen Arylrest mit bis zu 14 Kohlenstoffatomen, der gegebenenfalls durch eine oder mehrere funktionelle Gruppen substituiert ist, steht;
- den Resten 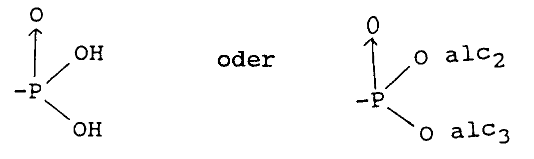 worin alc₂ und alc₃, die gleich oder verschieden voneinander sind, für einen linearen oder verzweigten, gesättigten oder ungesättigten Alkylrest mit bis zu 8 Kohlenstoffatomen, der gegebenenfalls durch eine oder mehrere funktionelle Gruppen substituiert ist, stehen, oder alc₂ und alc₃ mit dem Rest 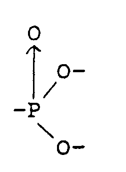 einen Zyklus 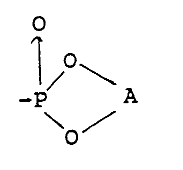 bilden können, worin A für eine lineare oder verzweigte, gesättigte oder ungesättigte, gegebenenfalls durch eine oder mehrere funktionelle Gruppen substituierte Kohlenstoffkette mit bis zu 6 Kohlenstoffatomen steht;
- den Resten 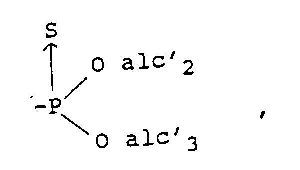 worin alc'₂ und alc'₃ wie alc₂ und alc₃ definiert sind;
- dem Rest 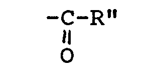 worin R" für einen Arylrest steht und R
- entweder für ein Wasserstoffatom,
- oder für einen linearen oder verzweigten, gegebenenfalls substituierten Alkylrest mit 1 bis 8 Kohlenstoffatomen,
- oder einen gegebenenfalls durch ein oder mehrere Reste, ausgewählt aus der Gruppe bestehend aus Alkylresten mit 1 bis 4 Kohlenstoffatomen, Alkenylresten mit 2 bis 6 Kohlenstoffatomen, Alkenyloxyresten mit 2 bis 6 Kohlenstoffatomen, Alkadienylresten mit 4 bis 8 Kohlenstoffatomen, dem Methylendioxyrest und den Halogenatomen substituierten Benzylrest;
- oder eine Gruppierung 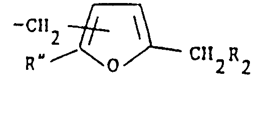 worin der Substituent R" für ein Wasserstoffatom oder einen Methylrest steht, und der Substituent R₂ für einen monocyclischen Arylrest oder eine -C=CH-Gruppe steht, und insbesondere eine 5-Benzyl-3-furylmethylgruppe,
- oder eine Gruppierung 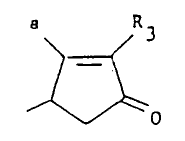 worin a für ein Wasserstoffatom oder einen Methylrest steht, und R₃ für einen aliphatischen organischen Rest mit 2 bis 6 Kohlenstoffatomen und einer oder mehreren ungesättigten Kohlenstoff-Kohlenstoff-Bindungen steht und insbesondere für die Gruppe -CH₂-CH=CH₂, -CH₂-CH=CH-CH₃, -CH₂-CH = CH-CH = CH₂, -CH₂-CH=CH-CH₂-CH₃, -CH₂-C=CH steht,
- oder eine Gruppierung 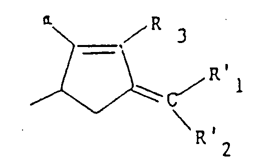 worin a für ein Wasserstoffatom oder einen Methylrest steht, R₃ wie vorstehend definiert ist, R'ᵢ und R'₂, die gleich oder verschieden sind, für ein Wasserstoffatom, ein Halogenatom, einen Alkylrest mit 1 bis 6 Kohlenstoffatomen, einen Arylrest mit 6 bis 10 Kohlenstoffatomen, eine Alkyloxycarbonylgruppierung mit 2 bis 5 Kohlenstoffatomen oder eine Cyanogruppe stehen,
- oder eine Gruppierung 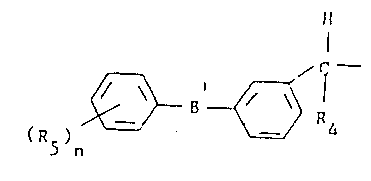 worin B' für ein Sauerstoff- oder Schwefelatom, eine 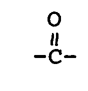 oder -CH₂-Gruppe oder eine Sulfoxidgruppe oder eine Sulfongruppe steht, und R₄ für ein Wasserstoffatom, einen -C=N-Rest, einen Methylrest, einen -CONH₂-Rest, einen -CSNH₂-Rest oder einen C = CH-Rest steht, R₅ für ein Halogenatom oder einen Methylrest steht, und n eine Zahl von 0, 1 oder 2 bedeutet, und insbesondere die 3-Phenoxybenzyl-, alpha-Cyano-3-phenoxybenzyl-, alpha-Ethinyl-3-phenoxybenzyl-, 3-Benzoylbenzyl-, 1-(3-Phenoxyphenyl)ethyl- oder alpha-Thioamido-3-phenoxybenzylgruppe,
- oder eine Gruppierung 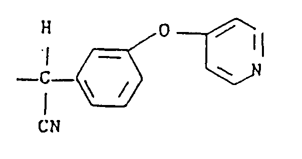
- oder eine Gruppierung 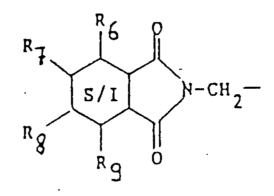 worin die Substituenten R₆, R₇, R₈, R₉ für ein Wasserstoffatom, ein Chloratom oder einen Methylrest stehen, und worin S/I einen aromatischen Zyklus oder einen anologen Dihydro- oder Tetrahydrozyklus bedeuten,
- oder eine Gruppierung 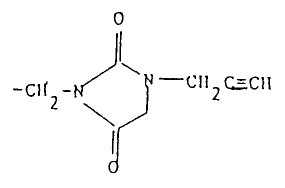
- oder eine Gruppierung 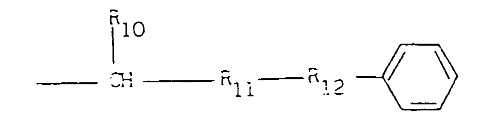 worin R₁₀ für ein Wasserstoffatom oder einen CN-Rest steht, R₁₂ für eine CH₂-Gruppe oder ein Sauerstoffatom steht, R₁₁ für einen Thiazolylrest oder Thiadiazolylrest steht, worin die Bindung mit 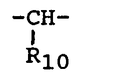 an irgendeiner verfügbaren Position sich befinden kann, wobei R_{12 an} R₁₁ durch das Kohlenstoffatom zwischen dem Schwefelatom und einem Stickstoffatom gebunden ist,
- oder eine Gruppierung 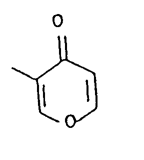
- oder eine Gruppierung 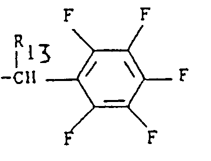 worin R₁₃ für ein Wasserstoffatom oder einen CN-Rest steht,
- oder eine Gruppierung 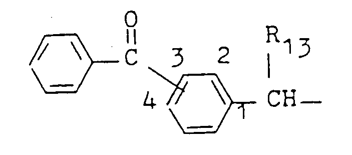 worin R_{13 3} wie vorstehend definiert ist, und der Benzoylrest sich in Position 3 oder 4 befindet,
- oder eine Gruppierung 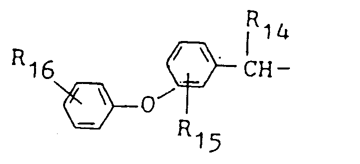 worin R₁₄ für ein Wasserstoffatom, eine Methyl-, Ethinyl- oder Cyanogruppe steht, und R₁₅ und R₁₆, die verschieden sind, für ein Wasserstoff-, Fluor- oder Bromatom stehen,
- oder eine Gruppierung 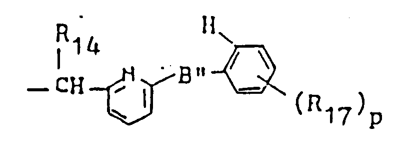 worin R₁₄ wie vorstehend definiert ist, worin jeder der Reste R₁₇ unabhängig für einen Alkylrest mit 1 bis 4 Kohlenstoffatomen, Alkoxyrest mit 1 bis 4 Kohlenstoffatomen, Alkylthiorest mit 1 bis 4 Kohlenstoffatomen, Alkylsulfonylrest mit 1 bis 4 Kohlenstoffatomen, Trifluormethylgruppe, 3,4-Methylendioxygruppe, Chlor-, Fluor- oder Bromatom steht, p eine Zahl 0, 1 oder 2 bedeutet, und B" für ein Sauerstoffatom oder ein Schwefelatom steht,
- oder eine Gruppierung 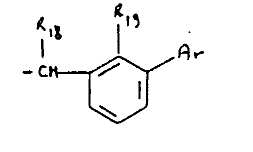 worin R₁₈ für ein Wasserstoffatom, eine Methyl-, Ethinyl- oder Cyanogruppe steht, und der sich unterscheidende Rest R₁₉ für ein Wasserstoff-, Fluor- oder Bromatom steht, und Ar für einen Arylrest mit bis zu 14 Kohlenstoffatomen steht,
- oder eine Gruppierung 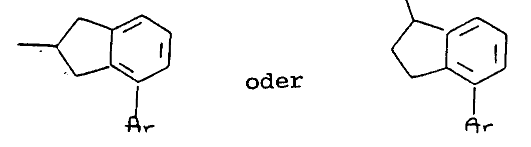
- oder eine Gruppierung 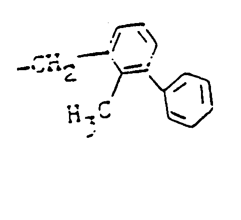
- oder eine Gruppierung 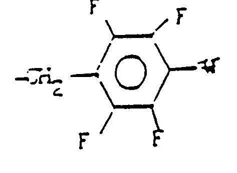 worin W = H, CH₃, CCH₃, steht, steht, mit der Maßgabe, daS R₁ keine CF₃-Gruppe bedeutet, wenn R=H, Alkyl, ausgenommen 2,2-Dimethyl-3-(1-methansulfonyloxy-2,2-dichlor-3,3,3-trifluorpropyl)cyclopropan-(2-methyl-3-phenylphenyl)methylcarboxylat, 2,2-Dimethyl-3-(1-tosyl-oxy-2,2-dichlor-3,3,3-trifluor-propylcyclopropan-(2-methyl-3-phenylphenyl)methylcarboxylat und 2,2-Dimethyl-3-(1-benzoyloxy-2,2-dichlor-3,3,3-trifluor- propylcyclopropan-(2-methyl-3-phenylphenyl)methylcarboxylat , dadurch gekennzeichnet, daß man eine Verbindung der vorstehend definierten Formel (I) der Wirkung eines Reduktionsmittels unterwirft, wodurch die entsprechende Verbindung der Formel (III) 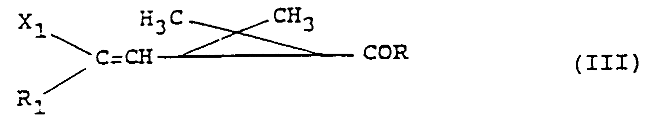 erhalten wird, worin X₁, R₁ und R wie vorstehend definiert sind.

2. Verwendung nach Anspruch 1, dadurch gekennzeichnet, daß Y für einen SO₂alc₁-Rest steht, worin alc₁ für einen Alkyl- oder Alkenylrest mit bis zu 8 Kohlenstoffatomen steht.

3. Verwendung nach Anspruch 2, dadurch gekennzeichnet, daß alc₁ für eine Methylgruppe steht.

4. Verwendung nach einem der Ansprüche 1 bis 3, dadurch gekennzeichnet, daß das Reduktionsmittel Wasserstoff in Gegenwart eines Hydrierungskatalysators, wie Palladium, ist.

5. Verwendung nach einem der Ansprüche 1 bis 4, dadurch gekennzeichnet, daß die Verbindung der Formel (I) der Formel (I') 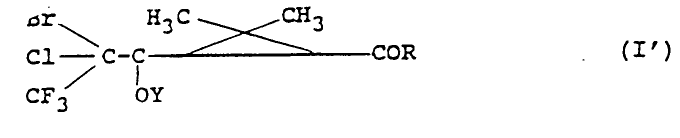 entspricht, worin Y und R wie vorstehend definiert sind, und die Verbindung der Formel (III) der Formel (III') 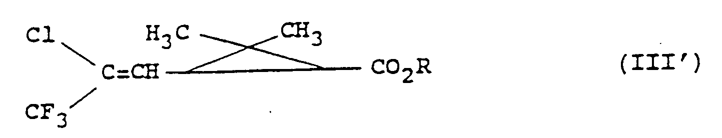 entspricht.

6. Verwendung nach einem der Ansprüche 1 bis 4, dadurch gekennzeichnet, daß die Verbindung der Formel (I) der Formel (I") 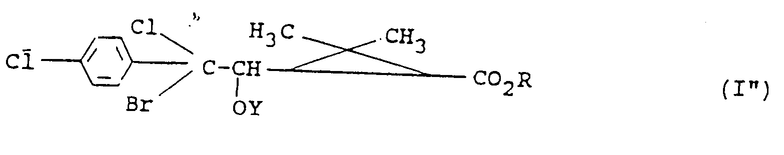 entspricht, und die erhaltene Verbindung der Formel (III) der Formel (III") 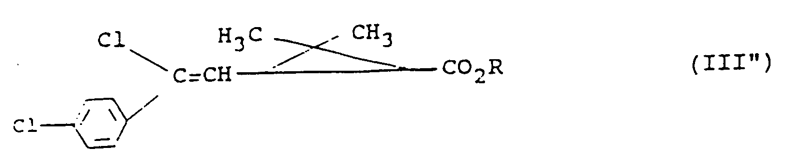 entspricht.
